# FASCICULE DE BREVET EUROPEEN

(11) **EP 1 668 094 B1**
(45) Date de publication et mention de la délivrance du brevet: **02.06.2010**
(21) Numéro de dépôt: 04787290.8
(22) Date de dépôt: 02.09.2004
(51) Int. Cl.: C10G 11/16, C07C 4/06

(54) **PROCEDE DE CONVERSION DIRECTE D'UNE CHARGE COMPRENANT DES OLEFINES A QUATRE, ET/OU CINQ ATOMES DE CARBONE OU PLUS, POUR LA PRODUCTION DE PROPYLENE**
VERFAHREN ZUR DIREKTUMWANDLUNG EINES OLEFINE MIT MINDESTENS VIER ODER FÜNF KOHLENSTOFFATOMEN ENTHALTENDEN EINSATZSTOFFS ZUR HERSTELLUNG VON PROPYLEN
METHOD FOR THE DIRECT CONVERSION OF A CHARGE CONTAINING OLEFINS COMPRISING A MINIMUM OF FOUR OR FIVE CARBON ATOMS, FOR PRODUCING PROPYLENE

(30) Priorité: 19.09.2003 FR 0311031
(43) Date de publication de la demande: 14.06.2006
(73) Titulaire: Institut Français du Pétrole, 92852 Rueil Malmaison Cedex (FR)
(72) Inventeur: DUPLAN, Jean-Luc, F-69540 Irigny (FR); LACOMBE, Sylvie, F-69230 St Genis Laval (FR)
(86) Numéro de dépôt international: PCT/FR2004/002235
(87) Numéro de publication internationale: WO 2005/028594

(56) Documents cités:
- EP-A- 0 921 179
- EP-A- 1 195 424
- WO-A-01/90034
- US-A1- 2003 139 636
- US-B1- 6 222 087

## Description

### Domaine de l'invention :

L'invention concerne un procédé permettant de convertir au moins en partie en propylène une charge d'hydrocarbures comprenant des oléfines dont le nombre de carbone est supérieur ou égal à 4, par exemple une coupe C4 et/ou C5 de vapocraquage ou de FCC. Le terme Cn indique une coupe d'hydrocarbures ayant essentiellement n atomes de carbone, et le terme FCC, abréviation de « Fluid Catalytic Cracking » dans la terminologie anglo-saxonne, signifie craquage catalytique (en lit) fluidisé. De façon générale, et selon la présente invention, le terme FCC désigne le procédé classique, utilisé en raffinerie, de craquage catalytique de fractions pétrolières lourdes, utilisant une charge bouillant principalement au dessus de environ 350 °C (au moins 50 % poids, généralement au moins 70 % poids et souvent 100 % poids de la charge bouillant au dessus de 350°C), par exemple du distillat sous vide, ou éventuellement de l'huile désasphaltée ou du résidu atmosphérique. Par extension, il désigne également les procédés similaires en lit fluidisé opérant à plus haute température, telle que environ 580°C à 700°C en sortie réacteur, et/ou utilisant des catalyseurs comprenant des zéolithes à forte réactivité comme la ZSM 5, pour obtenir une conversion poussée de la charge et une production accrue d'oléfines légères.

Ces coupes C4/C5 oléfiniques sont disponibles en quantité importante, souvent excédentaire, dans les raffineries de pétrole et les installations de vapocraquage. Or leur recyclage est problématique :
- leur recyclage au vapocraquage présente des inconvénients (les rendements en oléfines légères sont moins élevés qu' avec des coupes paraffiniques et leur tendance à la formation de coke est relativement plus élevée),
- leur recyclage au FCC n'est par ailleurs guère envisageable car elles sont très peu réactives dans les conditions du FCC, adaptées à la charge de distillat sous vide. Leur recyclage au FCC nécessiterait donc l'utilisation de conditions beaucoup plus sévères ou de catalyseurs spécifiques, ce qui modifierait de façon profonde le fonctionnement du FCC. Celui-ci ne pourrait être à la fois adapté au craquage de distillat sous vide et au craquage de coupes C4/C5.

La charge du procédé selon l'invention peut également comprendre des fractions C4/C5, ou plus larges provenant d'une unité de cokéfaction en chambre ou en lit fluidisé, ou de viscoréduction, ou de synthèse Fischer-Tropsch. La charge peut également comprendre des fractions d'une essence de vapocraquage ou d'une essence de FCC, ou d'une autre essence oléfinique. (On entend généralement par essence une coupe d'hydrocarbures issue pour sa plus grande partie au moins d'au moins une unité de conversion ou de synthèse (telle que FCC, viscoréduction, cokéfaction, unité Fischer-Tropsch, etc...) et dont la plus grande partie et typiquement au moins 90 % poids de cette coupe est constituée par des hydrocarbures ayant au moins 5 atomes de carbone et un point d'ébullition inférieur ou égal à environ 220°C).

La coupe oléfinique constituant la charge du procédé selon l'invention comprend de façon générale des oléfines ayant de 2 à 12 atomes de carbone. Elle est de préférence choisie parmi les charges définies précédemment, ou comprend un mélange des charges définies précédemment. Une charge typique comprend souvent des butènes et/ou des pentènes en quantité notable ou importante, et est typiquement constituée par une coupe oléfinique C4/C5. Elle peut comprendre également de l'éthylène, éventuellement de petites quantités de propylène non fractionné, des hexènes, des oléfines ayant de 7 à 10 atomes de carbone. Le plus souvent, la charge n'est pas purement oléfinique mais comprend aussi des paraffines (notamment du n-butane et/ou de l'iso-butane, des pentanes, et parfois des aromatiques notamment du benzène et/ou du toluène, et/ou des xylènes. Elle peut comprendre de l'isobutène et/ou des isoamylènes.

La charge comprend également souvent des composés fortement insaturés : diènes (dioléfines) à 4, 5 atomes de carbones notamment (en particulier du butadiène) et de petites quantités de composés acétyléniques ayant de 2 à 10 atomes de carbone.

La charge est donc typiquement une charge oléfinique légère, dont le point final de distillation (selon la méthode TBP, bien connue de l'Homme du métier), ou au moins le point pour lequel 90 % poids de la charge est distillée, est très généralement inférieur à 320°C, et généralement inférieur à 250°C.

Le procédé de conversion d'une charge oléfinique légère en une coupe comprenant du propylène, objet de la présente invention, met en oeuvre des réactions catalytiques permettant de convertir directement ces oléfines légères en propylène, c'est-à-dire sans étape préliminaire d'oligomérisation des oléfines.

L'installation pour mettre en oeuvre le procédé selon l'invention est de préférence installée à proximité ou sur un site de raffinage (raffinerie de pétrole), ou de pétrochimie (vapocraqueur).

### Art antérieur :

Un procédé connu de production directe de propylène, en dehors des procédés de production conventionnels que sont le FCC et le vapocraquage (ou le propylène est coproduit avec d'autres produits tels que notamment de l'essence ou de l'éthylène) est le procédé de métathèse qui convertit en propylène un mélange éthylène + n-butène. Ce procédé est décrit dans le brevet français FR 2 608 595.

Un des avantages du procédé selon l'invention par rapport à la métathèse, est de produire du propylène à partir de l'ensemble des composés oléfiniques des coupes C₄, C5 et éventuellement de coupes d'hydrocarbures de nombre d'atomes de carbone supérieur, notamment d'essence, et de ne pas requérir la consommation massive d'éthylène, produit de coût élevé. S'il est appliqué sur un site de vapocraquage, le procédé selon l'invention permet non seulement de ne pas utiliser d'éthylène comme charge, mais également de coproduire de l'éthylène avec le propylène. La coproduction d'éthylène étant typiquement inférieure à celle de propylène, ceci permet d'augmenter le ratio propylène sur éthylène du vapocraqueur.

Par ailleurs, s'il est appliqué en raffinerie de pétrole, le procédé selon l'invention permet au contraire de valoriser au besoin (en sus de coupes C4/C5) des quantités relativement limitées et donc difficilement valorisables d'éthylène, ce qui est souvent le cas en raffinerie.

On connaît également d'autres procédés de production de propylène à partir de coupes C4 et C5 oléfiniques en une seule étape de conversion chimique (c'est-à-dire sans oligomérisation préalable) :
Un autre procédé de production de propylène en une étape à partir d'oléfines légères est une variante du procédé FCC utilisant un catalyseur comprenant une zéolithe ZSM-5. Ce procédé est décrit dans la demande internationale WO 01/04237 ainsi que dans l'article « Maximizing Refinery Propylene Production Using ZSM-5 Technology », que l'on peut traduire par "Maximiser la production de propylène en raffinerie grâce à technologie ZSM-5" paru dans la revue "Hart's Fuel Technology and Management" ([revue Hart de] technologie et de gestion des combustibles), numéro de mai 1998. Les conditions opératoires typiques de ce procédé sont une température au voisinage de 600°C, une pression de 0,1 à 0,2 MPa.

Dans ces conditions, le rendement en propylène est d'environ 30 % et peut augmenter jusqu'à 50 % avec le recyclage des coupes C4 et C5 qui n'ont pas réagi.

Un inconvénient de ce procédé est que la technologie en lit fluidisé est relativement onéreuse du point de vue des investissements et nécessite une conduite du procédé relativement délicate. Elle conduit également à des pertes notables de catalyseur par attrition.

Dans la famille des procédés en une étape (sans oligomérisation préalable des fractions C4/C5), on peut également citer un procédé dont on trouvera une description dans l'article « Production Propylene from Low Valued Olefins » (Production de propylène à partir d'oléfines de faible valeur), paru dans la revue "Hydrocarbon Engineering" (Ingéniérie des hydrocarbures) de mai 1999. Il s'agit d'un procédé en lit fixe dont lé catalyseur est une zéolithe de type ZSM-5 agissant en présence de vapeur d'eau. La température est voisine de 500°C et la pression est comprise entre 0,1 et 0,2 MPa. La durée de cycle annoncée est de l'ordre de 1000 heures. Le catalyseur est régénéré in situ et sa durée de vie globale, c'est à dire la période pendant laquelle il est utilisé dans le réacteur avant son renouvellement complet, est d'environ 15 mois. Le rendement en propylène annoncé est de 40 % environ et pourrait monter à 60 % avec le recyclage des coupes C4 et C5 qui n'ont pas réagi. Ce procédé permet d'obtenir un rendement en propylène relativement élevé. Il requiert cependant l'utilisation de quantités élevées de vapeur d'eau.

On peut également citer un procédé décrit dans le brevet EP-B1-0 109 059. Il s'agit d'un procédé utilisant un catalyseur zéolithique de type ZSM-5 ou ZSM-11 ayant des caractéristiques particulières, utilisé avec une vitesse spatiale élevée. Des résultats d'essais, très vraisemblablement de courte durée et en lit fixe (microréacteur) sont indiqués.

On peut aussi citer un procédé décrit dans la demande internationale WO 99/29805 et dans les brevets ou demandes de brevet EP-0921181 et EP-A-0921179. Il s'agit d'un procédé utilisant un catalyseur zéolithique de type MFI ayant un rapport Si/Al élevé (de 180 à 1000) pour limiter les réactions de transfert d'hydrogène responsables de la production de diènes et d'aromatiques. La température est voisine de 550°C, la pression voisine de 0,1 MPa, et la vitesse spatiale comprise entre 10 h-1 et 30 h-1. Ce procédé mentionne la possibilité de réacteurs en lit fixe, mobile ou fluidisé. Il présente divers résultats d'essais expérimentaux, y compris d'essais de longue durée, et préconise l'utilisation d'un réacteur en lit fixe, ou plus exactement de deux réacteurs en lit fixe en alternance (en "swing" dans la terminologie anglo-saxonne), dont l'un est en opération et l'autre en régénération. Le catalyseur utilisé comporte une zéolithe de type MFI dont le rapport Si/Al (rapport atomique Silicium/Aluminium) est expressément supérieur ou égal à 180, et de préférence une zéolithe ZSM-5 de rapport Si/Al compris entre 300 et 1000.

L' utilisation de zéolithes désaluminisées de rapport Si/Al aussi élevés est préconisé et revendiqué tant pour limiter les réactions de transfert d'hydrogène que vis-à-vis de la stabilité de l'activité du catalyseur.

On peut enfin citer le procédé décrit dans la demande de brevet EP-A- 1 195 424 A1. Il s'agit d'un procédé utilisant aussi un catalyseur zéolithique de type MFI ayant un rapport Si/Al de 180 à 1000, ou un catalyseur zéolithique de type MEL ayant un rapport Si/Al de 150 à 800, ces rapports Si/Al élevés étant aussi utilisés pour limiter les réactions de transfert d'hydrogène responsables de la production de diènes et d'aromatiques. La température est comprise entre 500°C et 600°C, la pression partielle d'oléfines comprise entre 0,01 à 0,2 MPa, et la vitesse spatiale comprise entre 5 h-1 et 30 h-1. Ce procédé, qui représente l'art antérieur le plus proche de l'invention, utilise un lit mobile, avec prélèvement intermittent de catalyseur, qui est régénéré et recyclé.

### Description détaillée de l'invention :

Le demandeur a trouvé qu'il était possible de mettre en oeuvre un procédé présentant des avantages par rapport à l'art antérieur. Par rapport aux procédés en lit fixe (en swing) de l'art antérieur, le procédé selon l'invention permet d'obtenir une continuité de fonctionnement de l'unité de craquage (pouvant typiquement fonctionner dans le même réacteur pendant plus d'un, an voire beaucoup plus, sans arrêt de ce réacteur). Par rapport aux procédés en lit mobile, notamment le procédé décrit dans la demande de brevet EP-A- 1 195 424 A1, le procédé selon l'invention permet également d'obtenir une conversion et un rendement élevés mais avec un volume catalytique plus faible grâce notamment à l'utilisation d'un catalyseur adapté et d'une vitesse spatiale typiquement accrue.

La technologie de réacteur chimique opérant en lit catalytique granulaire mobile avec une boucle de régénération du catalyseur est une technologie bien connue dans l'industrie pétrolière et pétrochimique, et utilisée dans de nombreux procédés, par exemple dans les procédés de réformage catalytique continu d'hydrocarbures. On utilise typiquement un ou plusieurs réacteurs radiaux avec prélèvement continu ou généralement intermittent de catalyseur en partie basse de réacteur. On utilise par exemple un pot d'élévation ("lift pot" dans la terminologie anglo-saxonne) permettant de recueillir le catalyseur puis de le transférer par transport pneumatique, par exemple grâce à un courant d'azote, vers le réacteur suivant, ou la zone de régénération dans laquelle le catalyseur est régénéré par une ou plusieurs opérations comportant au moins une oxydation contrôlée des dépôts carbonés ou du coke par de l'air ou un gaz contenant de l'oxygène, par exemple de l'azote additionné d'air. Le catalyseur régénéré est alors transféré, par écoulement gravitaire ou par transport pneumatique vers la partie supérieure du réacteur, ou d'un autre réacteur. La zone de régénération peut opérer aussi en lit mobile, à une pression généralement voisine de la pression moyenne du procédé et à une température généralement comprise dans la gamme de 400°C à 650 °C. Le catalyseur peut, lorsqu'on utilise plusieurs réacteurs, circuler à contre-courant d'ensemble, ou à co-courant d'ensemble avec la charge. On pourra, pour obtenir d'autres détails sur les procédés en lit mobile, se référer notamment aux brevets US 3,838,039 , US 5,336,829 , US 5,849,976, et à la demande de brevet EP 1 195 424A1.

De façon plus précise, l'invention concerne un procédé de conversion directe (c'est-à-dire sans oligomérisation préalable des oléfines) par craquage catalytique d'une charge hydrocarbonée oléfinique légère comprenant au moins 80% poids d'hydrocarbures à au plus 12 atomes de carbone pour la production de propylène, ce procédé comprenant le craquage direct de la charge sur un catalyseur supporté comprenant au moins une zéolithe comprenant du silicium et de l'aluminium et présentant une sélectivité de forme, du groupe constitué d'une part par les zéolithes de l'un des types structuraux suivants :
MEL, MFI, NES, EUO, FER, CHA, MFS, MWW, et d'autre part par les zéolithes suivantes: NU-85, NU-86, NU-88 et IM-5, dans lequel on fait circuler la charge à une température comprise entre environ 480°C et 620°C dans au moins un réacteur sur un lit mobile granulaire dudit catalyseur, on soutire, en continu ou en discontinu en partie inférieure du réacteur un débit de catalyseur comprenant un dépôt carboné, on transfère ce catalyseur dans une zone de régénération ou il est soumis à au moins une étape d'oxydation contrôlée, puis, en aval de la zone de régénération, l'on réintroduit le catalyseur comprenant un taux réduit de dépôt carboné (par rapport au catalyseur soutiré), directement ou indirectement, en partie supérieure dudit réacteur,
Les catalyseurs utilisables selon l'invention sont tels que les zéolithes du groupe précité peuvent présenter selon différentes variantes de l'invention un rapport atomique Si/Al pouvant varier dans une large gamme. Ils présentent par exemple un rapport Si/Al typiquement compris entre 20 et 1500. Cependant, selon l'une des variantes préférées de l'invention, les zéolithes de ce groupe présentent un rapport Si/Al inférieur à 160, par exemple une ou plusieurs zéolithes du sous-groupe MFI (par exemple de la ZSM-5), de rapport Si/Al inférieur à 160, notamment compris entre 30 et 160.

De façon générale, on peut utiliser avantageusement une ou plusieurs zéolithes du groupe (le plus large) précité qui présentent un rapport Si/Al inférieur à 130, par exemple compris entre 40 et 130, ou entre 60 et 120, et en particulier une ou plusieurs zéolithes du sous-groupe constitué par les zéolithes de type structural MFI, MEL, CHA, avec de tels rapports Si/Al.

Typiquement, au moins 80% poids de la charge est issue directement d'une ou plusieurs unités de craquage d'hydrocarbures, par exemple d'unités du groupe des unités FCC, vapocraquage, viscoréduction, cokéfaction.

La charge peut aussi comprendre des fractions issues directement d'une ou plusieurs unités de synthèse Fischer-Tropsch, par exemple au moins 10 % poids de telles fractions.

De préférence, la ou les zéolithes dudit groupe appartiennent au sous groupe constitué par les zéolithes de type structural MEL, MFI et CHA, ou au sous-groupe des zéolithes de type structural MFI. On peut notamment utiliser une zéolithe ZSM-5.

Typiquement, le procédé permet d'utiliser une vitesse spatiale VVH globale (par rapport à l'ensemble des zones réactionnelles ou réacteurs) comprise généralement entre 7 et 100, de préférence entre 13 et 80, ou même comprise entre 33 et 60 ce qui permet de réduire encore plus le volume catalytique. Selon l'une des variantes préférées de l'invention, on utilise en effet un catalyseur plus actif que ceux de l'art antérieur décrits dans le brevet EP-0921181 et dans la demande de brevet EP-A- 1 195 424 A1. On utilise donc avantageusement un rapport Si/Al relativement faible, avec une vitesse spatiale accrue, en limitant le temps de contact pour que la réaction de craquage vers le propylène s'approche de l'équilibre thermodynamique sans que les réactions de transfert d'hydrogène ne continuent à progresser. La vitesse spatiale élevée permet également, à débit de catalyseur constant, de réduire le temps de séjour dans le réacteur. L'utilisation d'un catalyseur de Si/Al plus bas, donc d'activité accrue, conjointement avec une VVH augmentée permet d'obtenir une réduction du temps de résidence du catalyseur dans la zone réactionnelle globale, et donc de contrecarrer l'augmentation de la vitesse de cokéfaction du catalyseur du fait du transfert d'hydrogène accru résultant du rapport Si/Al plus faible. On obtient ainsi un nouvel équilibre des variables opératoires avec un volume réactionnel pouvant être notablement réduit par rapport à celui de l'art antérieur.

Selon une autre variante préférée de l'invention, utilisable avec les faibles rapports Si/Al précités, mais également avec des rapports Si/Al très élevés, par exemple entre 130 et 1200 voire plus, on utilise un lit mobile à vitesse de circulation atypique, avec notamment des temps de résidence dans le réacteur (ou dans la zone réactionnelle globale, si l'on utilise plusieurs zones réactionnelles ou plusieurs réacteurs) pouvant être compris entre 1 et 40 heures, et de préférence entre 2 et 18 heures. Ces valeurs sont très basses par rapport aux lits mobiles conventionnels utilisés par exemple pour le réformage catalytique régénératif ou les temps de résidence globaux (pour l'ensemble des réacteurs) sont typiquement de 2 à 3 jours. Elles peuvent être obtenues en utilisant des VVH élevées et en maintenant des taux de prélèvement importants de catalyseur coké envoyé vers la zone de régénération. L'utilisation de taux de soutirage élevés permet d'obtenir une réduction supplémentaire du temps de résidence du catalyseur dans la zone réactionnelle globale, et donc de contrecarrer encore l'augmentation de la vitesse de cokéfaction du catalyseur du fait du transfert d'hydrogène accru résultant du rapport Si/Al souvent plus faible.

Pour réduire encore le transfert d'hydrogène, on peut selon l'une des variantes préférées de l'invention, et quel que soit le rapport Si/Al, avantageusement diluer la charge, notamment à raison de 10 à 70% molaire de diluant, par un mélange d'hydrogène et de méthane, et plus particulièrement par un tel mélange issu du train de fractionnement des effluents d'un vapocraqueur. Ceci est notamment intéressant si le vapocraqueur est situé sur le même site que l'unité de craquage catalytique en lit mobile selon l'invention, qui craque une partie des fractions légères (C4/C5 notamment) produites par le vapocraqueur. On peut aussi utiliser de la vapeur d'eau (seule ou avec le mélange d'hydrogène et de méthane) pour abaisser la pression partielle des hydrocarbures.

Selon l'une des mises en oeuvre préférées du procédé selon l'invention, la charge traverse en série de 2 ou 3 zones réactionnelles en lit mobile avec réchauffage intermédiaire entre 2 zones successives. On peut utiliser plusieurs réacteurs ou plusieurs zones réactionnelles d'un même réacteur. La vitesse spatiale VVH dans chaque zone réactionnelle peut être comprise par exemple entre 14 et 160, notamment entre 66 et 120. On peut utiliser éventuellement une vitesse spatiale plus élevée dans le premier réacteur.

La charge avant d'être introduite dans l'unité de craquage en lit mobile peut de préférence subir préalablement une hydrogénation sélective, dans une étape préliminaire afin d'éliminer les dioléfines et autres impuretés acétyléniques souvent présentes dans la charge. Ces différents composés fortement insaturés contribuent en effet à une certaine désactivation du catalyseur de craquage et l'hydrogénation sélective permet d'augmenter la quantité d'oléfines convertibles.

L'effluent de craquage catalytique est typiquement soumis à une étape de fractionnement comprenant le plus souvent une compression des gaz et une ou plusieurs distillations pour séparer les effluents et produire une coupe C3 riche en propylène, ou du propylène sensiblement pur.

Si l'unité de craquage catalytique selon le procédé de l'invention est située sur le même site qu'une unité de vapocraquage, ou bien qu'une unité FCC, les effluents de craquage catalytique peuvent être réunis avec ceux de vapocraquage ou du FCC, pour être fractionnés en commun. Du fait de la moindre réactivité des paraffines en craquage catalytique, ceci pourra conduire à augmenter la quantité de paraffines n'ayant pas réagi dans la fraction oléfinique récupérée, principalement composée d'effluents de vapocraquage, qui est envoyée au craquage catalytique selon l'invention. Pour éviter un gonflement excessif de ce volant de paraffines on pourra éliminer par une purge une partie de la charge oléfinique, par exemple de la coupe C5/C6, cette purge n'étant pas recyclée au craquage catalytique selon l'invention. La purge peut être recraquée par recyclage aux fours de vapocraquage.

On peut aussi traiter et séparer les effluents de craquage catalytique en lit mobile séparément de ceux de vapocraquage, ou de FCC.

On décrit ci-après plus en détail les conditions particulières des différentes étapes réactionnelles du procédé selon l'invention, selon une variante comprenant une hydrogénation sélective puis un craquage catalytique en lit mobile, intégrés sur un même site, la charge utilisée étant une coupe légère d'hydrocarbures en C4 et C5 contenant principalement des butènes, des pentènes, des butanes, des pentanes ainsi que dans certains cas, du butadiène et du pentadiène en quantité variable.

### 1) Hydrogénation sélective (étape optionnelle préférée) :

La coupe légère provient typiquement d'un craqueur catalytique FCC et/ou d'un vapocraqueur. Les teneurs en diènes et en acétyléniques sont importantes quand cette coupe vient d'un vapocraqueur ; c'est pourquoi l'étape d'hydrogénation sélective des diènes et des acétyléniques en oléfines est quasiment indispensable dans ce cas. Elle est également préférable dans la plupart des cas, car elle réduit le cokage du catalyseur de craquage et accroît la conversion en propylène. On ne sortirait toutefois pas du cadre de l'invention si une telle étape d'hydrogénation sélective n'est pas comprise dans le procédé selon l'invention.

L'objet principal de cette première étape est de transformer les dioléfines (ou diènes) en mono-oléfines. En effet, les mono-oléfines sont la source des molécules réactives en craquage catalytique. Le deuxième objet de cette étape est d'éliminer les traces d'hydrocarbures acétyléniques toujours présentes dans ces coupes et qui sont des composés indésirables pour le craquage catalytique, favorisant le cokage du catalyseur.

Ces composés sont également transformés en mono-oléfines.

Lorsque la proportion de dioléfines dans la coupe est importante, on peut avantageusement effectuer la transformation dans deux, ou trois réacteurs en série afin de mieux contrôler la sélectivité de l'hydrogénation. Souvent on dilue la charge à traiter par recyclage d'un certain débit de l'effluent de cette hydrogénation sélective.

La teneur résiduelle en dioléfines + acétyléniques de l'effluent de l'hydrogénation sélective est typiquement inférieure à environ 1000 ppm poids, de préférence inférieure à environ 100 ppm poids et de façon très préférée inférieure à 20 ppm poids. La teneur résiduelle en acétyléniques peut même être inférieure à 10 ppm, ou 5 ppm, ou même à 1 ppm poids.

La quantité d'hydrogène nécessaire à l'ensemble des réactions réalisées dans cette étape est généralement ajustée en fonction de la composition de la coupe pour avoir avantageusement seulement un léger excès d'hydrogène par rapport à la stoechiométrie.

Généralement, cette étape d'hydrogénation sélective est réalisée en utilisant un catalyseur comprenant au moins un métal choisi dans le groupe formé par le nickel, le palladium, et le platine, déposé sur un support comprenant de l'alumine, de la silice ou de la silice-alumine. On met en oeuvre de préférence un catalyseur qui comprend au moins du palladium ou un composé de palladium fixé sur un support minéral réfractaire, par exemple sur une alumine ou une silice-alumine. La teneur en palladium sur le support peut être typiquement de 0,01 % à 5 % en poids, de préférence de 0,05 % à 1 % en poids. Divers modes de prétraitement connus de l'homme du métier peuvent éventuellement être appliqués à ces catalyseurs pour améliorer leur sélectivité d'hydrogénation vers les mono-oléfines.

La température opératoire de l'hydrogénation sélective est généralement comprise entre 0 et 200°C, la pression typiquement comprise entre 0,1 et 5 MPa, souvent entre 0,5 et 5 MPa, la vitesse spatiale typiquement comprise entre 0,5 et 20 m³ par heure et par m³ de catalyseur, souvent entre 0,5 et 5 m³ par heure et par m³ de catalyseur, et le rapport molaire H2/(composés acétyléniques + dioléfiniques) généralement compris entre 0,5 et 5 et de préférence entre 1 et 3.

On utilise généralement, pour réaliser l'hydrogénation sélective un réacteur en lit fixe, traversé à co-courant descendant par la charge à traiter et de l'hydrogène, ou à courant descendant pour la charge à traiter et à courant ascendant pour de l'hydrogène.

On peut également réaliser une ou plusieurs étapes éventuelles de purification de la charge (par exemple désulfuration, et/ou de séchage, et/ou de déazotation, et/ou de déoxygénation) en amont du craquage catalytique, si nécessaire, en fonction de la charge et du catalyseur utilisés.

L'étape d'hydrogénation sélective est essentielle dans le cas de coupes oléfiniques de vapocraquage (notamment de coupes C4/C5), en raison de la teneur très élevée en butadiène.

On peut aussi extraire l'isobutène avant le craquage catalytique en lit mobile selon l'invention.

L'extraction de l'iso-butène peut être réalisée par distillation extractive, par exemple avec un solvant qui peut être La N-methyl pyrrolidone (NMP) ou le Di-méthyl sulfoxyde ( DMSO) ou un isomère de ce dernier.

L'extraction de l'iso-butène, et éventuellement d'autres oléfines branchées, notamment des isoamylènes, peut également comprendre une éthérification de l'isobutène par un alcool, puis une distillation. On peut également réaliser une hydroisomérisation avec distillation réactive, pour séparer l'isobutène du butène (le butène 1 étant transformé en butène 2 séparable de l'isobutène).

### 2) Craquage catalytique :

La charge alimentée au craquage catalytique en lit mobile contient typiquement de 20 à 100 % poids, souvent de 25 à 60 % poids d'oléfines, notamment d'oléfines légères à 4 et/ou 5 atomes de carbone.

Typiquement, le catalyseur peut comprendre au moins une zéolithe présentant une sélectivité de forme, cette zéolithe comprenant du silicium et au moins un élément choisi dans le groupe formé par l'aluminium, le fer, le gallium, le phosphore, le bore, et de préférence l'aluminium. Cette zéolithe présentant une sélectivité de forme peut être de l'un des types structuraux suivants : MEL (par exemple la ZSM-11), MFI (par exemple la ZSM-5), NES, EUO, FER, CHA (par exemple la SAPO-34), MFS, MWW, ou peut également être l'une des zéolithes suivantes: NU-85, NU-86, NU-88 et IM-5, qui présentent également une sélectivité de forme.

L'un des avantage de ces zéolithes présentant une sélectivité de forme est qu'il conduit à une meilleure sélectivité propylène / isobutène (rapport propylène / isobutène plus élevé dans les effluents de craquage).

On peut également utiliser plusieurs zéolithes présentant une sélectivité de forme, par exemple une zéolithe de type MFI (par exemple la ZSM-5) associée à une autre zéolithe présentant une sélectivité de forme, précédemment citée ou de l'un des types précédemment cités.

La (ou les) zéolithe(s) présentant une sélectivité de forme, du groupe formé par les zéolithes de l'un des types structuraux suivants : MEL (par exemple la ZSM-11), MFI (par exemple la ZSM-5), NES, EUO, FER, CHA (par exemple la SAPO-34), MFS, MWW, ou du groupe des zéolithes suivantes: NU-85, NU-86, NU-88 et IM-5, peut également être mélangée avec une zéolithe ne présentant pas de sélectivité de forme, telle que par exemple une zéolithe Y de type structural FAU.

Souvent on utilise un catalyseur comprenant une ou plusieurs zéolithe(s) présentant une sélectivité de forme, la proportion de zéolithe(s) présentant une sélectivité de forme étant comprise entre 70 et 100 % poids, bornes comprises, par rapport à la quantité totale de zéolithe(s). On peut notamment utiliser un catalyseur dont la proportion de zéolithe(s) présentant une sélectivité de forme est comprise entre 80 et 100 % poids par rapport à la quantité totale de zéolithe(s), et même un catalyseur dont la ou les zéolithes présentent toutes une sélectivité de forme.

La ou les zéolithes peuvent être dispersées dans une matrice à base de silice, zircone, alumine ou de silice alumine, la proportion de zéolithe (et généralement de zéolithe présentant une sélectivité de forme) étant souvent comprise entre 15 et 80 % poids, de préférence entre 30 et 75 % poids, par exemple entre 40 et 75 % poids. La matrice est de préférence choisie de façon à être peu acide, ou non acide. On pourra notamment utiliser une matrice à taux d'alumine réduit ou nul, par exemple de la silice et/ou de la zircone.

La zéolithe utilisée (ou les zéolithes utilisées) présentant une sélectivité de forme présente(nt) de façon préférée selon l'invention un rapport Si/Al relativement faible, par exemple inférieur à 300 ou même inférieur à 130. La synthèse de zéolithes est un domaine bien connu de l'homme du métier. Des exemples de synthèse sont donnés par exemple dans les brevets ou demandes de brevet US 3,702,886 (ZSM-5 exemple 24), US 3,709,979 (ZSM-11), FR 2 755 958 et EP-A2-463 768 (NU-86), EP-0921181 et EP-A-0921179 (ZSM-5,) et EP-A-1 195 424 A1 (ZSM-5 et ZSM-11). On pourra trouver d'autres informations sur les zéolithes dans: "Atlas of zeolithes structure types" (atlas des types de structure des zéolithes) par Meier WM et Olsen DH, 1992, publié par Butterworths, zeolithes vol 2, No 15, juin 1992, et des méthodes de synthèse dans "Synthesis of high silica aluminosilicates zeolithes" (synthèse de zéolithes aluminosilicates de haute teneur en silice) par PA Jacobs et JA Martens, studies in surface science and catalysis (Etudes dans la science des surfaces et des catalyseurs), vol 33, Elsevier, 1987.

Des rapports Si/Al variables, modérés ou élevés peuvent être obtenus au moment de la fabrication de la zéolithe, ou par désalumination et élimination d'alumine ultérieure. On peut notamment utiliser l'une des zéolithes commerciales ZSM-5 : CBV 28014 (rapport Si/Al 140), et CBV 1502 (rapport Si/Al 75) de la société Zeolyst International, Valley Forge PA., 19482 USA, ou la ZSM-5 Pentasil de rapport Si/Al 125 de Sud-Chemie (Munich, Allemagne).

On peut aussi utiliser, à titre d'exemple la méthode suivante de préparation d'une zéolithe ZSM-5 de rapport Si/Al 120:

La composition du gel utilisé pour la synthèse est définie dans le tableau 1.

**Tableau 1 : Composition du gel**

| | |
|---|---|
| SiO₂ (mol) | 60 |
| Al₂O₃ (mol) | 0,3 |
| Na₂O (mol) | 10 |
| TPABr (mol) | 7 |
| H₂O (mol) | 2000 |

| | |
|---|---|
| TPABr = bromure de tétrapropylamonium | |

On prépare la solution A composée de l'eau, de l'hydroxyde de sodium solide (Prolabo) et du bromure de tétrapropylammonium (Fluka). Une solution B est préparée en ajoutant à la solution A de l'hydroxyde d'aluminium (Reheis Ireland) sous agitation. On mélange à température pendant environ quinze minutes. On ajoute ensuite à la solution B la silice (Ludox HS40, Dupont de Nemours) sous agitation. On mélange à température ambiante jusqu'à homogénéisation, soit environ une heure. On fait réagir le mélange résultant dans un autoclave sous agitation pendant 4 jours à 175°C sous pression autogène. Après refroidissement, on filtre le produit et on le lave avec de l'eau déminéralisée puis on le sèche en étuve ventilée à 120°C. Le solide est ensuite calciné à 550°C sous air pendant 5h afin d'éliminer le structurant organique. Puis le solide obtenu est soumis à trois échanges ioniques dans une solution de NH₄NO₃ 10N à environ 100°C pendant 4 heures pour chaque échange.

Une analyse par diffraction des rayons X réalisée sur le produit obtenu montre que le produit est constitué de zéolithe MFI pure bien cristallisée. L'analyse élémentaire par fluorescence X permet de mesurer un rapport Si/Al molaire de 123. La teneur pondérale en sodium par rapport au poids de zéolithe MFI sèche est de 85 ppm.

La zéolithe est soumise à un vapotraitement à 600°C sous 50%vol de vapeur d'eau dans de l'azote pendant 5 heures.

Pour la fabrication du catalyseur, on peut utiliser des techniques classiques, par exemple mélanger la zéolithe avec des précurseurs de gel de silice et/ou des gels de silice, puis mettre en forme sous forme de petites billes par coagulation en gouttes, sècher puis calciner les billes pour obtenir le catalyseur final.

Si l'on part d'une zéolithe dont le rapport Si/Al est inférieur à la valeur voulue, on peut, pour augmenter ce rapport, procéder à une désalumination suivie par un traitement d'élimination d'alumine amorphe. Le traitement de désaluminisation, qui permet d'éliminer de l'aluminium de la structure zéolithique peut être conduit sous atmosphère de vapeur d'eau sous une pression de 20 à 200 kPa, à une température comprise entre 500 et 800 °C, et une durée comprise entre 1 et 200 heures. On ajuste l'intensité du traitement (durée, température, ou pression de vapeur d'eau) en fonction du rapport initial Si/Al de la zéolithe et du rapport final désiré. On élimine alors l'alumine amorphe ainsi extraite de la structure zéolithique par un traitement postérieur d'extraction avec formation d'un complexe d'aluminium soluble dans l'eau. On peut trouver d'autres éléments pour la réalisation de ces traitements, ou la fabrication de catalyseurs de craquage, dans les brevets ou demandes de brevet WO 99/29805, EP-0921181 et EP-A-0921179.

Le catalyseur est mis en oeuvre en lit mobile, sous forme de billes (préférentiellement) ou d'extrudés de diamètre généralement compris entre 0,4 et 6 mm, de préférence entre 0,6 et 4 mm.

La phase de régénération comprend typiquement une phase de combustion des dépôts carbonés formés sur le catalyseur, par exemple à l'aide d'un mélange air/azote ou d'air appauvri en oxygène (par exemple par recirculation de fumées), ou d'air, et peut éventuellement comprendre d'autres phases de traitement et de régénération du catalyseur.

On peut aussi selon l'une des variantes préférées de l'invention, utilisable quel que soit le rapport Si/Al du catalyseur, limiter le taux de régénération du catalyseur pour que le rapport du taux de dépôt carboné du catalyseur prélevé (usé) sur le taux de dépôt carboné du catalyseur régénéré soit relativement faible, par exemple compris entre 1,1 et 10, de préférence entre 1,2 et 6. Les rapports les plus préférés sont compris entre 1,2 et 4, et même entre 1,3 et 3, voire entre 1,3 et 2,5. Ceci permet de ne pas avoir des activités catalytiques trop éloignées entre le catalyseur usé et le catalyseur régénéré, celui-ci augmentant trop le transfert d'hydrogène et la saturation des oléfines. Cette limitation de la régénération peut être obtenue aisément, par ajustement (réduction) du débit de gaz oxydant, et/ou de la température d'oxydation et/ou de la pression partielle d'oxygène. On peut aussi piloter le débit de soutirage de catalyseur usé, ou ajuster le temps de résidence du catalyseur dans le lit mobile, notamment dans les intervalles de durée précités.

On opère habituellement le craquage catalytique à une température d'environ 450 à environ 650°C et de préférence entre 480°C et 620°C avec une vitesse spatiale comprise généralement entre 7 et 80 h⁻¹, notamment entre 13 et 80 h⁻¹, ou même entre 33 et 60 h⁻¹. La pression opératoire est généralement comprise entre 0,1 et 5 MPa, le plus souvent comprise entre 0,1 et 1,5 MPa, et de préférence entre 0,1 et 0,5 MPa.

Les conditions de régénération du catalyseur de craquage utilisent généralement une température comprise entre 300 et 900°C, notamment entre 500 et 750°C, la pression étant le plus souvent voisine de la pression de craquage, ou bien proche de la pression atmosphérique.

Généralement, le rendement en propylène rapporté à la quantité d'oléfines contenues dans la charge fraîche du procédé est compris entre 30 % et 60 % poids, et souvent entre 40 et 60 % poids.

Le procédé selon l'invention n'est pas limité aux éléments précédemment décrits, et peut être mis en oeuvre selon des variantes ou avec des modes de réalisation non décrits dans la présente description mais déjà connus de l'homme du métier.

## Revendications

1. - Procédé de conversion directe par craquage catalytique d'une charge hydrocarbonée oléfinique légère comprenant au moins 80 % poids d'hydrocarbures à au plus 12 atomes de carbone pour la production de propylène, ce procédé comprenant le craquage direct de la charge sur un catalyseur supporté comprenant au moins une zéolithe comprenant du silicium et de l'aluminium et présentant une sélectivité de forme, du groupe constitué d'une part par les zéolithes
de l'un des types structuraux suivants : MEL, MFI, NES, EUO, FER, CHA, MFS, MWW, et d'autre part par les zéolithes suivantes: NU-85, NU-86, NU-88 et IM-5, dans lequel on fait circuler la charge à une température comprise entre environ 480°C et 620°C dans au moins un réacteur sur un lit mobile granulaire dudit catalyseur, on soutire, en continu ou en discontinu, en partie inférieure du réacteur un débit de catalyseur comprenant un dépôts carboné, on transfère ce catalyseur dans une zone de régénération ou il est soumis à au moins une étape d'oxydation contrôlée, puis, en aval de la zone de régénération, l'on réintroduit le catalyseur comprenant un taux réduit de dépôt carboné, directement ou indirectement, en partie supérieure dudit réacteur,
le catalyseur utilisé étant tel que les zéolithes dudit groupe présentent un rapport Si/Al compris entre 40 et 130, et ledit procédé étant tel que la vitesse spatiale VVH globale est comprise entre 13 et 80 h⁻¹.

2. - Procédé selon la revendication 1, dans lequel au moins 80% poids de la charge est issue directement d'une ou plusieurs unités de craquage d'hydrocarbures.

3. - Procédé selon l'une des revendications 1 et 2, dans lequel au moins 10% poids de la charge est issue directement d'une ou plusieurs unités de synthèse Fischer-Tropsch.

4. - Procédé selon l'une des revendications 1 à 3, dans lequel la ou les zéolithes dudit groupe appartiennent au sous groupe constitué par les zéolithes de type structural MEL, MFI et CHA.

5. - Procédé selon l'une des revendications 1 à 4, dans lequel la ou les zéolithes dudit groupe sont de type structural MFI.

6. - Procédé selon l'une des revendications 1 à 5, dans lequel la ou les zéolithes dudit groupe sont constitués par de la zéolithe ZSM-5.

7. - Procédé selon l'une des revendications 1 à 6, dans lequel la vitesse spatiale VVH globale est comprise entre 33 et 60 h⁻¹.

8. - Procédé selon l'une des revendications 1à 7, dans lequel le temps de résidence du catalyseur dans la zone réactionnelle est compris entre 1 et 40 heures.

9. - Procédé selon l'une des revendications 1 à 8, dans lequel le temps de résidence du catalyseur dans la zone réactionnelle est compris entre 2 et 18 heures.

## Claims

1. A process for direct conversion, by catalytic cracking, of a light olefinic hydrocarbon feed comprising at least 80% by weight of hydrocarbons containing at most 12 carbon atoms for the production of propylene, said process comprising direct cracking of the feed on a supported catalyst comprising at least one zeolite comprising silicon and aluminium and having form selectivity, from the group constituted by zeolites with one of the following structure types: MEL, MFI, NES, EUO, FER, CHA, MFS, MWW, and from the following zeolites: NU-85, NU-86, NU-88 and IM-5, comprising circulating the feed at a temperature in the range about 480°C to 620°C in at least one reactor on a granular moving bed of said catalyst, extracting from the lower portion of the reactor, continuously or discontinuously, a flow of catalyst comprising a carbonaceous deposit, transferring said catalyst to a regeneration zone where it undergoes at least one controlled oxidation step, then, downstream of the regeneration zone, re-introducing the catalyst comprising a reduced amount of carbonaceous deposit directly or indirectly into the upper portion of said reactor, the catalyst used being such that the zeolites from said group have a Si/Al ratio in the range 40 to 130, said process being such that the overall space velocity HSV is in the range 13 to 80 h⁻¹.

2. A process according to claim 1, in which at least 80% by weight of the feed is derived directly from one or more hydrocarbon cracking units.

3. A process according to claim 1 or claim 2, in which at least 10% by weight of the feed is derived directly from one or more Fischer-Tropsch synthesis units.

4. A process according to one of claims 1 to 3, in which the zeolite or zeolites of said group belong to the sub-group constituted by zeolites of structure type MEL, MFI and CHA.

5. A process according to one of claims 1 to 4, in which the zeolite or zeolites of said group are of structure type MFI.

6. A process according to one of claims 1 to 5, in which the zeolite or zeolites of said group are constituted by ZSM-5 zeolite.

7. A process according to one of claims 1 to 6, in which the overall space velocity is in the range 33 to 60 h⁻¹.

8. A process according to one of claims 1 to 7, in which the residence time for the catalyst in the reaction zone is in the range 1 to 40 hours.

9. A process according to one of claims 1 to 8, in which the residence time for the catalyst in the reaction zone is in the range 2 to 18 hours.

## Patentansprüche

1. Verfahren zur direkten Umwandlung durch katalytisches Cracken einer leichten olefinischen Kohlenwasserstoffbeschickung, die mindestens 80 Gew.-% Kohlenwasserstoffe mit höchstens 12 Kohlenstoffatomen umfasst, zur Produktion von Propylen, wobei dieses Verfahren das direkte Cracken der Beschickung auf einem geträgerten Katalysator umfasst, der mindestens einen Zeolithen, der Silicium und Aluminium umfasst und eine Formselektivität aufweist, der Gruppe bestehend einerseits aus den Zeolithen
einer der folgenden Strukturtypen: MEL, MFI, NES, EUO, FER, CHA, MFS, MWW und andererseits aus den folgenden Zeolithen: NU-85, NU-86, NU-88 und IM-5 umfasst, wobei die Beschickung bei einer Temperatur im Bereich zwischen etwa 480 °C und 620 °C in mindestens einem Reaktor auf einem granulären Wanderbett des Katalysators zirkuliert wird, im unteren Teil des Reaktors ein Strom aus Katalysator, der eine kohlenstoffhaltige Abscheidung umfasst, kontinuierlich oder diskontinuierlich entnommen wird, dieser Katalysator in eine Regenerationszone transportiert wird, wo er mindestens einem Schritt der kontrollierten Oxidation unterzogen wird, der Katalysator, der einen reduzierten Anteil an kohlenstoffhaltiger Abscheidung umfasst, dann stromabwärts der Regenerationszone direkt oder indirekt wieder in den oberen Teil des Reaktors einführt wird,
wobei der verwendete Katalysator so ist, dass die Zeolithen der Gruppe ein Si/Al-Verhältnis im Bereich zwischen 40 und 130 aufweisen und das Verfahren so ist, dass die globale Raumgeschwindigkeit HSV im Bereich zwischen 13 und 80 h⁻¹ liegt.

2. Verfahren nach Anspruch 1, wobei mindestens 80 Gew.-% der Beschickung direkt aus einer oder mehreren Einheiten zum Cracken von Kohlenwasserstoffen stammen.

3. Verfahren nach einem der Ansprüche 1 und 2, wobei mindestens 10 Gew.-% der Beschickung direkt aus einer oder mehreren Einheiten zur Fischer-Tropsch-Synthese stammen.

4. Verfahren nach einem der Ansprüche 1 bis 3, wobei der oder die Zeolithen der Gruppe zur Untergruppe bestehend aus den Zeolithen vom Strukturtyp MEL, MFI und CHA gehören.

5. Verfahren nach einem der Ansprüche 1 bis 4, wobei der oder die Zeolithen der Gruppe vom Strukturtyp MFI sind.

6. Verfahren nach einem der Ansprüche 1 bis 5, wobei der oder die Zeolithen der Gruppe aus dem Zeolithen ZSM-5 bestehen.

7. Verfahren nach einem der Ansprüche 1 bis 6, wobei die globale Raumgeschwindigkeit HSV im Bereich zwischen 33 und 60 h⁻¹ liegt.

8. Verfahren nach einem der Ansprüche 1 bis 7, wobei die Verweildauer des Katalysators in der Reaktionszone im Bereich zwischen 1 und 40 Stunden liegt.

9. Verfahren nach einem der Ansprüche 1 bis 8, wobei die Verweildauer des Katalysators in der Reaktionszone im Bereich zwischen 2 und 18 Stunden liegt.
